# EUROPÄISCHE PATENTSCHRIFT

(11) **EP 0 661 264 B1**
(45) Veröffentlichungstag und Bekanntmachung des Hinweises auf die Patenterteilung: **12.07.2000**
(21) Anmeldenummer: 94120773.0
(22) Anmeldetag: 27.12.1994
(51) Int. Cl.: C07D 207/38, A61K 31/40

(54) **Substituierte Benzolsulfonylharnstoffe und -thioharnstoffe als Antiarrhythmika**
Substituted benzenesulfonylureas and -thioureas as antiarrhythmics
Benzènesulfonylurées et -thiourées comme agents antiarrhythmiques

(30) Priorität: 30.12.1993 DE 4344957
(43) Veröffentlichungstag der Anmeldung: 05.07.1995
(73) Patentinhaber: HOECHST AKTIENGESELLSCHAFT, 65929 Frankfurt am Main (DE)
(72) Erfinder: Englert, Heinrich, Dr., D-65719 Hofheim (DE); Mania, Dieter, Dr., D-61462 Königstein (DE); Hartung, Jens, Dr., D-97204 Höchberg (DE); Gögelein, Heinz, Dr., D-60259 Frankfurt (DE); Kaiser, Joachim, Dr., D-60431 Frankfurt (DE); Gerlach, Uwe, Dr., D-65795 Hattersheim (DE)

(56) Entgegenhaltungen:
- EP-A- 0 031 058
- CHEMICAL ABSTRACTS, vol. 111, no. 21, 20. November 1989, Columbus, Ohio, US; abstract no. 186728g, FOSSET,M. ET AL. 'Identification, mechanism of function and regulation of ATP-sensitive potassium channels as targets for sulfonylureas used in the treatment of Type II diabetes' & JOURN.ANNU. DIABETOL. HOTEL-DIEU 1989,7-11

## Beschreibung

Substituierte Benzolsulfonylharnstoffe und -thioharnstoffe, Herstellungsverfahren und Verwendungsmöglichkeiten pharmazeutischer Präparate auf Basis dieser Verbindungen

Die Erfindung betrifft substituierte Benzolsulfonylharnstoffe und -thioharnstoffe der Formel I 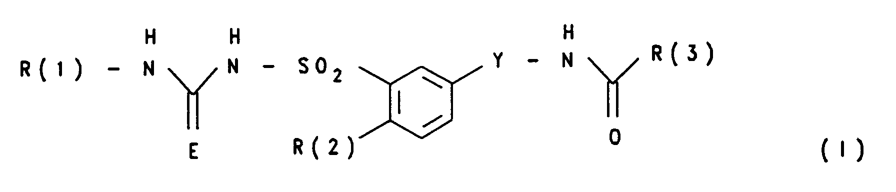 worin bedeuten:
- R(1): (C₁-C₆)-Alkyl, (C₃-C₆)-Cycloalkyl, CₐH₂ₐ-(C₃-C₅)-Cycloalkyl oder C_{c}H_{2c}-C_{d}F_{2d+1};
a 1, 2 oder 3;
c 0, 1, 2 oder 3;
d 1, 2, 3, 4, 5 oder 6;
- R(2): F, Cl, Br, I, (C₁-C₆)-Alkyl, (C₁-C₆)-Alkoxy, (C₁-C₆)-Mercaptoalkyl, (C₁-C₆)-Fluoralkoxy, (C₁-C₆)-Fluoralkyl, (C₃-C₆-Cycloalkyl oder NR(4)R(5);
R(4) und R(5) gemeinsam eine (CH₂)₄₋₆-Kette, in der eine der CH₂-Gruppen durch Sauerstoff, Schwefel oder NR(6) ersetzt sein kann, wobei zwischen dem N-Atom des NR(4)R(5) und dem Sauerstoff, Schwefel oder NR(6) mindestens eine CH₂-Gruppe stehen muß;
   oder
R(4), R(5), R(6) unabhängig voneinander Wasserstoff, (C₁-C₆)-Alkyl, (C₃-C₆)-Cycloalkyl oder (C₃-C₆)-Alkenyl;
- E: Sauerstoff oder Schwefel;
- Y: eine Kohlenwasserstoffkette -[CR(7)₂]ₙ-;
R(7) H oder (C₁-C₂)-Alkyl;
n 1, 2, 3 oder 4;
- R(3): einen Heterocyclus der Formel 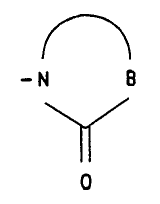
B (C₃-C₆)-Alkenylen, das unsubstituiert oder mit bis zu 3 (C₁-C₄)-Alkylgruppen oder mit einem Phenylrest substituiert ist;
und ihre physiologisch unbedenklichen Salze.

Bevorzugt sind Verbindungen der Formel I, in welcher bedeuten:
- R(1): (C₁-C₄)-Alkyl, (C₃-C₅)-Cycloalkyl oder C_{c}H_{2c}-C_{d}F_{2d+1};
c 0, 1, 2 oder 3;
d 1, 2, 3, 4, 5 oder 6;
- R(2): F, Cl, (C₁-C₆)-Alkyl, (C₁-C₆)-Alkoxy, (C₁-C₆)-Mercaptoalkyl, (C₁-C₆)-Fluoralkoxy, (C₁-C₆)-Fluoralkyl, (C₃-C₆)-Cycloalkyl oder NR(4)R(5);
R(4) und R(5) gemeinsam eine (CH₂)₄₋₆-Kette, in der eine der CH₂-Gruppen durch Sauerstoff, Schwefel oder NR(6) ersetzt sein kann, wobei zwischen dem N-Atom des NR(4)R(5) und dem Sauerstoff, Schwefel oder NR(6) mindestens eine CH₂-Gruppe stehen muß;
   oder
R(4), R(5), R(6) unabhängig voneinander Wasserstoff, (C₁-C₆)-Alkyl, (C₃-C₆)-Cycloalkyl oder (C₃-C₆)-Alkenyl;
- E: Sauerstoff oder Schwefel;
- Y: eine Kohlenwasserstoffkette -[CR(7)₂]ₙ-;
R(7) H oder (C₁-C₂)-Alkyl;
n 1, 2, 3 oder 4;
- R(3): einen Heterocyclus der Formel 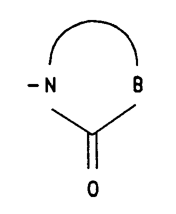
B (C₃-C₆)-Alkenylen, das unsubstituiert oder mit bis zu 3 (C₁-C₄)-Alkylgruppen oder mit einem Phenylrest substituiert ist;
und ihre physiologisch unbedenklichen Salze.

Besonders bevorzugt sind Verbindungen der Formel I, in welcher bedeuten:
- R(1): (C₁-C₄)-Alkyl, (C₃-C₄)-Cycloalkyl oder (C₁-C₄)-Fluoralkyl;
- R(2): F, Cl, (C₁-C₄)-Alkyl, (C₁-C₄)-Fluoralkyl oder (C₃-C₆)-Cycloalkyl;
- E: Sauerstoff oder Schwefel;
- Y: eine Kohlenwasserstoffkette -[CR(7)₂]ₙ-;
R(7) H oder (C₁-C₂)-Alkyl;
n 2 oder 3;
- R(3): einen Heterocyclus der Formel 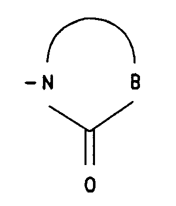
B (C₃-C₆)-Alkenylen, das unsubstituiert oder mit bis zu 3 (C₁-C₄)-Alkylgruppen oder mit einem Phenylrest substituiert ist;
und ihre physiologisch unbedenklichen Salze.

Ebenfalls besonders bevorzugt sind Verbindungen der Formel I, in welcher bedeuten:
- R(1): (C₁-C₄)-Alkyl, (C₃-C₄)-Cycloalkyl oder (C₁-C₄)-Fluoralkyl;
- R(2): (C₁-C₄)-Alkoxy, (C₁-C₄)-Mercaptoalkyl oder (C₁-C₄)-Fluoralkoxy;
- E: Sauerstoff oder Schwefel;
- Y: eine Kohlenwasserstoffkette -[CR(7)₂]ₙ-;
R(7) H oder (C₁-C₂)-Alkyl;
n 2 oder 3;
- R(3): einen Heterocyclus der Formel 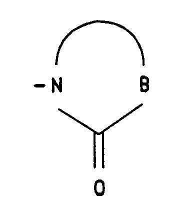
B (C₃-C₄)-Alkenylen, das unsubstituiert oder mit ein bis drei (C₁-C₄)-Alkylgruppen oder mit einem Phenylrest substituiert ist;
und ihre physiologisch unbedenklichen Salze.

Weiterhin besonders bevorzugt sind Verbindungen der Formel I, in welcher bedeuten:
- R(1): (C₁-C₄)-Alkyl oder (C₁-C₄)-Fluoralkyl;
- R(2): NR(4)R(5);
R(4) und R(5) gemeinsam eine (CH₂)₄₋₆-Kette, in der eine der CH₂-Gruppen durch Sauerstoff, Schwefel oder NR(6) ersetzt sein kann, wobei zwischen dem N-Atom des NR(4)R(5) und dem Sauerstoff, Schwefel oder NR(6) mindestens eine CH₂-Gruppe stehen muß; oder
R(4), R(5) unabhängig voneinander CH₃, C₂H₅, n-C₃H₇, Iso-C₃H₇ oder Cyclo-C₃H₅;
R(6) H, CH₃ oder C₂H₅;
- E: Sauerstoff oder Schwefel;
- Y: eine Kohlenwasserstoffkette -[CR(7)₂]ₙ-;
R(7) H oder (C₁-C₂)-Alkyl;
n 2 oder 3;
- R(3): einen Heterocyclus der Formel 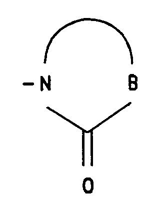
B (C₃-C₄)-Alkenylen, das unsubstituiert oder mit bis zu 3 (C₁-C₄)-Alkylgruppen oder mit einem Phenylrest substituiert ist;
und ihre physiologisch unbedenklichen Salze.

Der Begriff Alkyl beschreibt, sofern nicht anders angegeben, geradkettige oder verzweigte gesättigte Kohlenwasserstoffreste. Der Cycloalkylrest kann zusätzlich einen Alkylsubstituenten tragen.

Fluoralkyl und Fluoralkoxy bedeutet, sofern nicht ausdrücklich anders angegeben, einen geradkettigen oder verzweigten gesättigten Alkyl- oder Alkoxyrest mit 1 bis 6 C-Atomen, in welchem mindestens ein oder höchstens alle Wasserstoffe durch Fluor ersetzt sind.

Weiterhin können Verbindungen mit Chiralitätszentren in der Alkylseitenkette Y auftreten. In diesem Fall gehören sowohl die einzelnen Antipoden für sich als auch eine Mischung der beiden Enantiomeren in unterschiedlichen Verhältnissen sowie die dazugehörigen Mesoverbindungen oder Mischungen aus Mesoverbindungen, den Enantiomeren oder Diastereomeren zur Erfindung.

Ähnliche Sulfonylharnstoffe sind aus der Deutschen Offenlegungsschrift 2 413 514, der deutschen Patentschrift 1 518 874 und der Europäischen Patentschrift 0031058 bekannt. Die DE-OS 2 413 514 und die EP 0031058 beschreiben ausschließlich Blutzucker-konditionierende Stoffe mit p-Substitution in der zentralen Phenylgruppe. Hinweise auf m-Substitution finden sich nicht.

In den Patent-Veröffentlichungen werden die blutzuckersenkenden Wirkungen der Sulfonylharnstoffe beschrieben. Als Prototyp solcher blutzuckersenkenden Sulfonylharnstoffe gilt das Glibenclamid, das als Mittel zur Behandlung des Diabetes mellitus therapeutisch verwendet wird und in der Wissenschaft ein vielbeachtetes Werkzeug zur Erforschung sogenannter ATP sensitiver Kaliumkanäle dient. Neben seiner blutzuckersenkenden Wirkung besitzt das Glibenclamid noch andere Wirkungen, die bislang therapeutisch noch nicht eingesetzt werden können, die aber allesamt auf Blockade eben dieser ATP-sensitiven Kalium-Kanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann.

Aufgabe der vorliegenden Erfindung war es daher, Verbindungen zu synthetisieren, die eine gleich gute Herzwirkung wie Glibenclamid aufweisen aber den Blutzucker in herzwirksamen Dosen oder Konzentrationen nicht oder deutlich geringer beeinflussen als Glibenclamid.

Als Versuchstiere zum Nachweis solcher Wirkungen eignen sich zum Beispiel Mäuse, Ratten, Meerschweinchen, Kaninchen, Hunde, Affen oder Schweine.

Die Verbindungen I dienen als Arzneimittelwirkstoffe in der Human- und Veterinärmedizin. Ferner können sie als Zwischenprodukte zur Herstellung weiterer Arzneimittelwirkstoffe verwendet werden.

Die Verbindungen I der vorliegenden Erfindung sind wertvolle Arzneimittel zur Behandlung von Herzrhythmusstörungen unterschiedlichster Genese und zur Verhinderung des arrhythmisch bedingten, plötzlichen Herztodes und können daher als Antiarrhythmika Verwendung finden. Beispiele arrhythmischer Störungen des Herzens sind supraventrikuläre Rhythmusstörungen wie etwa Vorhof-Tachykardien, Vorhofflattern oder paroxysmale supraventrikuläre Rhythmusstörungen, oder ventrikuläre Rhythmusstörungen, wie ventrikuläre Extrasystolen, insbesondere aber lebensbedrohende ventrikuläre Tachykardien oder das besonders gefährliche Kammerflimmern. Sie eignen sich insbesondere für solche Fälle, in denen Arrhythmien Folge einer Verengung eines Koronargefäßes sind, wie sie beispielsweise bei Angina Pectoris oder während eines akuten Herzinfarktes oder als chronische Folge eines Herzinfarktes auftreten. Sie sind daher insbesondere bei Postinfarktpatienten zur Verhinderung des plötzlichen Herztodes geeignet. Weitere Krankheitsbilder, wo derartige Rhythmusstörungen und/oder der plötzliche, arrhythmisch bedingte Herztod eine Rolle spielen sind beispielsweise die Herzinsuffizienz oder die Herzhypertrophie als Folge eines chronisch erhöhten Blutdruckes.

Darüber hinaus können die Verbindungen I eine verminderte Kontraktilität des Herzens positiv beeinflussen. Hierbei kann es sich um ein krankheitsbedingtes Nachlassen der Herzkontraktilität handeln, beispielsweise bei Herzinsuffizienz aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch kardioplegische Lösungen erforderlich machen, wobei die Verbindungen sowohl für den Schutz der Organe beispielsweise bei Behandlung mit oder deren Lagerung in physiologischen Badflüssigkeiten, wie auch bei der Überführung in den Empfängerorganismus verwendet werden können.

Die Erfindung betrifft weiterhin ein Verfahren zur Herstellung der Verbindungen I dadurch gekennzeichnet, daß man
(a) Sulfonamide der Formel II oder deren Salze der Formel III
   mit R(1)-substituierten Isocyanaten der Formel IV

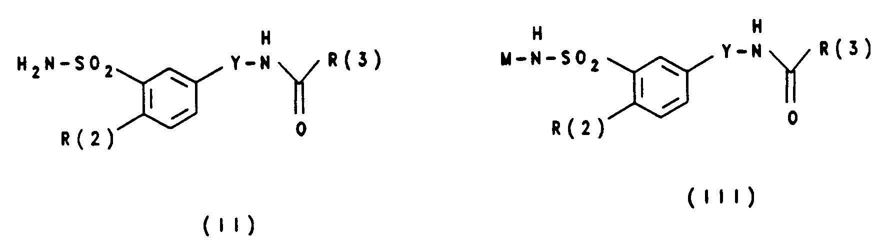

   R(1) - N = C = O IV

   zu substituierten Benzolsulfonylharnstoffen I a umgesetzt.
   Als Kationen M in den Salzen der Formel III kommen Alkali- und Erdalkaliionen in Betracht, insbesondere Na⁺, K⁺, Ca⁺⁺,Mg⁺⁺. Äquivalent zu den R(1)-substituierten Isocyanaten IV kann man R(1)-substituierte Carbamidsäureester, R(1)-substituierte Carbamidsäure-halogenide oder R(1)-substituierte Harnstoffe einsetzen.
(b) Benzolsulfonylharnstoffe I a 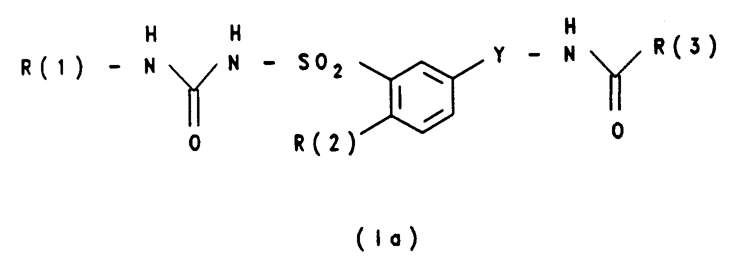 lassen sich aus aromatischen Benzolsulfonamiden II oder deren Salze III und aus R(1)-substituierten Trichloracetamiden der Formel V 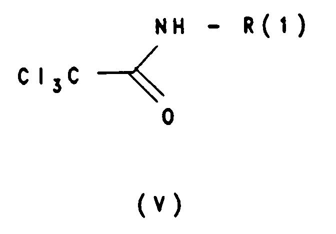 in Gegenwart einer Base in einem inerten Lösungsmittel nach Synthesis 1987, 734 - 735 bei Temperaturen von 25°C bis 150°C darstellen.
   Als Basen eignen sich zum Beispiel Alkalimetall- oder Erdalkalimetallhydroxide, oder auch -alkoholate, wie Natriumhydroxid, Kaliumhydroxid, Calciumhydroxid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Nitrile wie Acetonitril, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(c) Benzolsulfonylthioharnstoffe I b 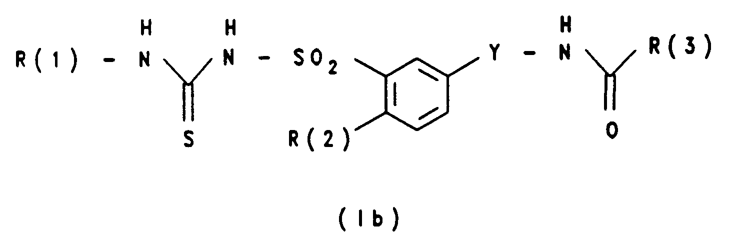 werden aus Benzolsulfonamiden II sowie deren Salze III und R(1)-substituierten Thioisocyanaten VI

   R(1) - N = C = S VI

   dargestellt.
(d) Substituierte Benzolsulfonylharnstoffe der Formel I a können durch Umwandlungsreaktionen von Benzolsulfonylthioharnstoffen der Struktur I b dargestellt werden. Der Ersatz des Schwefelatoms durch ein Sauerstoffatom in den entsprechend substituierten Benzolsulfonylthioharnstoffen I b kann man beispielsweise mit Hilfe von Oxiden oder Salzen von Schwermetallen oder durch auch durch Anwendung von Oxidationsmitteln wie Wasserstoffperoxid, Natriumperoxid oder Salpetersäure ausgeführt werden. Thioharnstoffe können auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Benzolsulfonylharnstoffe I a überführt werden. Isothioharnstoffe verhalten sich bei der Entschwefelung wie Thioharnstoffe und können demzufolge ebenso als Ausgangsstoffe für diese Reaktionen dienen.
(e) Benzolsulfonylharnstoffe I a lassen sich durch Umsetzungen von Aminen der Formel R(1)-NH₂ mit Benzolsulfonylisocyanaten der Formel VII 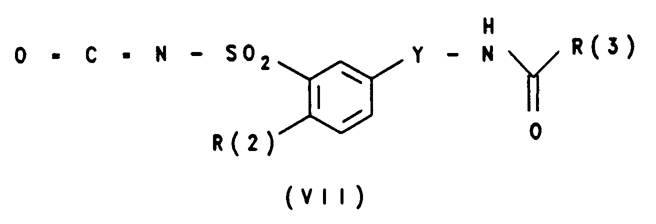 herstellen. Ebenso können Amine R(1)-NH₂ mit Benzolsulfonylcarbamidsäureestern, -carbamidsäurehalogeniden oder Benzolsulfonylharnstoffen I a (mit R(1) = H) zu den Verbindungen I a umgesetzt werden.
(f) Benzolsulfonylthioharnstoffe I b lassen sich durch Umsetzungen von Aminen der Formel R(1)-NH₂ mit Benzolsulfonylisothiocyanaten der Formel VIII 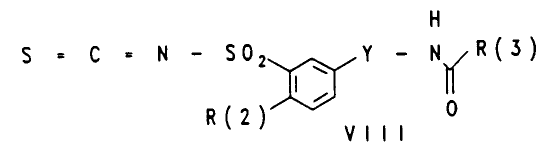 herstellen. Ebenso können Amine R(1)-NH₂ mit Benzolsulfonylcarbamidsäurethioestern, -carbamidsäurethiohalogeniden zu den Verbindungen I b umgesetzt werden.

Die Verbindungen I sowie deren physiologisch unbedenkliche Salze sind wertvolle Therapeutika, die sich nicht nur als Antiarrhythmika, sondern als Prophylaxe bei Störungen des kardiovaskulären Systems, Herzinsuffizienz, Herztransplantation oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remington's Pharmaceutical Sciences, 17. Auflage, 1985, Seite 1418 Verbindungen der Formel IX, 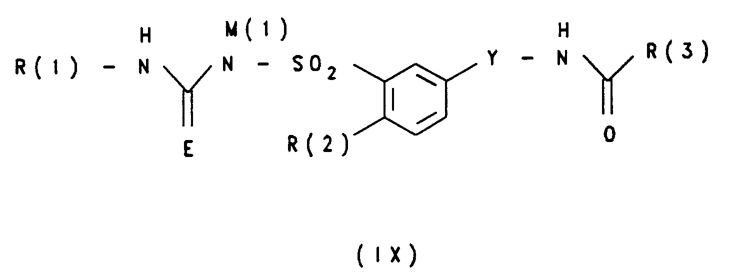 die sich aus nicht toxischen organischen und anorganischen Basen und Benzolsulfonylharnstoffen I darstellen lassen.

Bevorzugt werden hierbei Salze in denen M(1) in der Formel IX Natrium-, Kalium-, Rubidium-, Calcium-, Magnesiumionen sind, sowie die Säureadditionsprodukte basischer Aminosäuren, wie zum Beispiel Lysin oder Arginin.

Die Ausgangsverbindungen für die erwähnten Syntheseverfahren der Benzolsulfonylharnstoffe I werden nach an sich bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

Das Amin X wird zunächst in ein Isocyanat oder ein reaktives Kohlensäurederivat überführt (Schema 1). Die Umwandlung des Amins X in ein Isocyanat erfolgt in bekannter Weise durch Reaktion von X mit Kohlensäurehalogeniden wie Phosgen oder Triphosgen in Gegenwart tertiärer Alkylamine oder Pyridin und inerter Solventien. Als inerte Lösungsmittel eignen sich Ether wie Tetrahydrofuran, Dioxan, Ethylenglykoldimethylether (Diglyme), Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander. Als reaktive Kohlensäurederivate kommen Kohlensäureester in Betracht, wie sie sich aus Chlorameisensäurealkylestern und X sowie geeigneter tertiärer Alkylamine oder Pyridin synthetisieren lassen. Weiterhin können N,N-Carbonyldiimidazol und analoge reaktive Derivate als Isocyanatäquivalente eingesetzt werden (Staab, H.A.: Synthesen mit heterocyclischen Amiden (Azoliden). In: Angewandte Chemie 74 (1962), Nr. 12, S. 407-423).

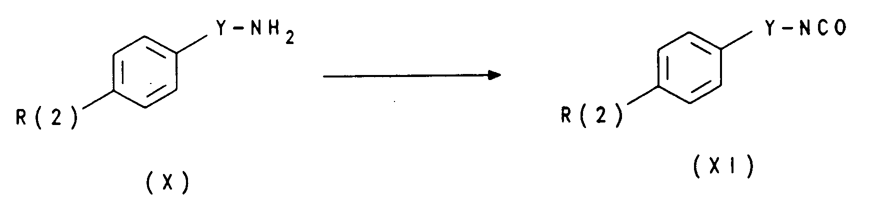

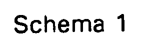

Das Isocyanat XI oder entsprechende Urethane werden mit der zweiten Molekülkomponente (Justus Liebigs Ann. chem. 1956, 598, S. 203) in An- oder Abwesenheit inerter Lösungsmittel bei Temperaturen von 100 - 170°C gekoppelt und liefern die Acylharnstoff-Derivate XII (Schema 2)

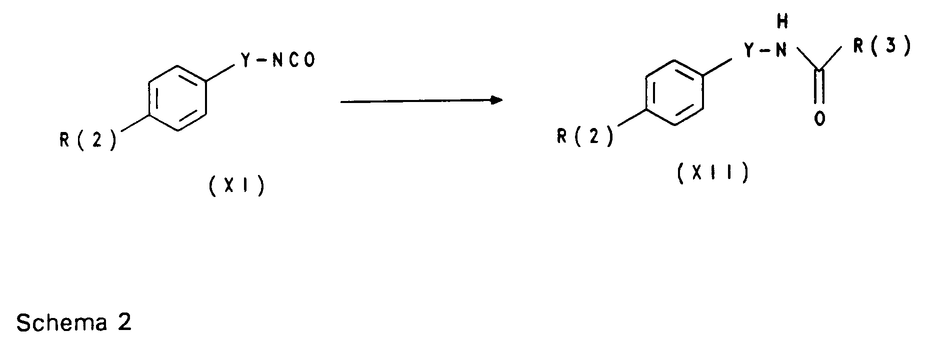

Die nach Schema 2 synthetisierten Verbindungen XII können in bekannter Weise nach Schema 3 in die Sulfonamide II überführt werden. Die Sulfonamide II werden nach an sich bekannten Methoden hergestellt und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Synthesen können gewünschtenfalls in einem, zwei oder mehreren Schritten vollzogen werden. Insbesondere sind Verfahren bevorzugt, in denen das Acylharnstoff-Derivat XII durch elektrophile Reagenzien in An- oder Abwesenheit von inerten Lösungsmitteln bei Temperaturen zwischen -10°C bis 120°C, vorzugsweise zwischen 0°C und 100°C in aromatische Sulfonsäuren sowie deren Derivate wie zum Beispiel Sulfonsäurehalogenide überführt wird. Beispielsweise können Halogensulfonierungen mit Halogensulfonsäuren durchgeführt werden. Dabei können ortho und meta Isomere entstehen, die sich nach Standardverfahren (zum Beispiel Säulenchromatographie an inerten Trägermaterialien oder Kristallisation aus inerten Lösungsmitteln) auf der nächstfolgenden Sulfonamidreaktionsstufe voneinander trennen lassen. Die Überführung der Sulfonsäurederivate in Sulfonamide II erfolgt in literaturbekannter Weise, vorzugsweise werden Sulfonsäurechloride in inerten Lösungsmitteln bei Temperaturen von 0°C bis 100°C mit wäßrigem Ammoniak umgesetzt.

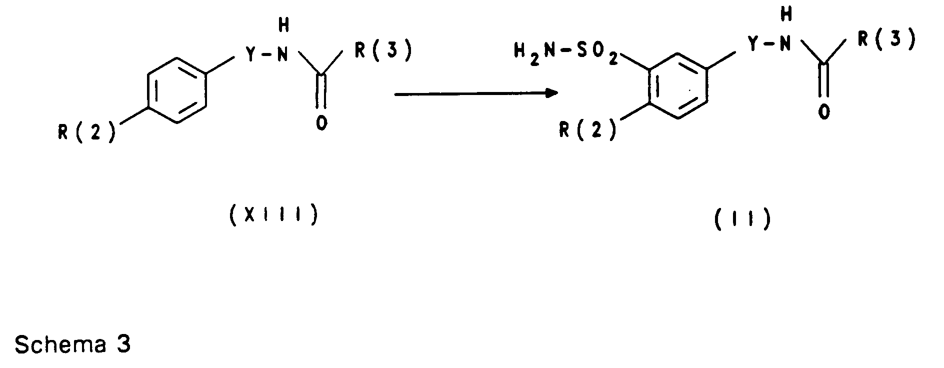

Aus den so hergestellten sulfonamidsubstituierten Acylharnstoff-Derivaten II oder deren Säureadditionsverbindungen, werden wie oben erwähnt die Benzolsulfonylharnstoffe der Formel I hergestellt. Je nach der Natur der Glieder R(1), R(2), R(3), E, B, X, Y und Z wird in einzelnen Fällen das eine oder andere der genannten Verfahren für die Herstellung der Verbindungen I ungeeignet sein oder zumindest Vorkehrungen zum Schutz aktiver Gruppen notwendig machen. Derartige, verhältnismäßig selten auftretende Fälle können vom Fachmann unschwer erkannt werden, und es bereitet keine Schwierigkeiten, in solchen Fällen einen anderen der beschriebenen Synthesewege erfolgreich anzuwenden.

Die Verbindungen I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomere, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäuren, Mandelsäuren, Äpfelsäure oder Milchsäure. Carbinole können ferner mit Hilfe chiraler Acylierungsreagenzien, zum Beispiel R- oder S-α-Methylbenzylisocyanat amidiert und dann getrennt werden. Die verschiedenen Formen der Diastereomeren können in an sich bekannter Weise, zum Beispiel durch fraktionierte Kristallisation, getrennt und die Enantiomeren der Formel I können in an sich bekannter Weise aus den Diastereomeren in Freiheit gesetzt werden. Enantiomerentrennungen gelingen ferner durch Chromatographie an optischaktiven Trägermaterialien.

Die erfindungsgemäßen Verbindungen I und ihre physiologisch unbedenklichen Salze können zur Herstellung pharmazeutischer Zubereitungen verwendet werden. Hierbei können sie zusammen mit mindestens einem festen, flüssigen Träger oder Hilfsstoff allein oder in Kombination mit anderen Herz-Kreislaufaktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen, wie Ethanol oder Isopropanol, Acetonitril, 1,2-Propandiol oder deren Gemische untereinander oder mit Wasser) oder Puder. Die neuen Verbindungen können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht. Die Stabilisierungs- und/oder Netzmittel, Emulgatoren, Salze und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks- und/oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von mindestens etwa 0.001 mg, vorzugsweise etwa 0.01 mg, insbesondere etwa 0.1 mg, bis höchstens etwa 100 mg, vorzugsweise etwa 10 mg, insbesondere etwa 1 mg pro kg und Tag aus, wenn Prophylaxe betrieben wird. Bevorzugt ist ein Dosisbereich von 1 bis 10 mg pro kg und Tag. Die Dosis kann dabei als orale oder parenterale Einzeldosis oder in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Ein bevorzugte Dosisbereich in kritischen Situationen kann dann 10 bis 100 mg betragen und beispielsweise als intravenöse Dauerinfusion verabreicht werden.

Erfindungsgemäß können außer den in den Ausführungsbeispielen beschriebenen Beispielen die in der folgenden Tabelle zusammengestellten Verbindungen I erhalten werden:

### 3-Ethyl-4-methyl-2-oxo-3-pyrrolinyl-1-carboxamide:

(1) 1-{2-Methoxy-5-[2-(3-ethyl-4-methyl-2-oxo-3-pyrrolinyl-1-carboxamido)-ethyl]-phenylsulfonyl-}3-ethylharnstoff,
(2) 1-{2-Methoxy-5-[2-(3-ethyl-4-methyl-2-oxo-3-pyrrolinyl-1-carboxamido)-ethyl]-phenylsulfonyl-}3-(1-propyl)-harnstoff,
(3) 1-{2-Methoxy-5-[2-(3-ethyl-4-methyl-2-oxo-3-pyrrolinyl-1-carboxamido)-ethyl]-phenylsulfonyl-}3-(2-propyl)-harnstoff,
(4) 1-{2-Methoxy-5-[2-(3-ethyl-4-methyl-2-oxo-3-pyrrolinyl-1-carboxamido)-ethyl]-phenylsulfonyl-}3-(cyclopropyl)-harnstoff,
(5) 1-{2-Ethoxy-5-[2-(3-ethyl-4-methyl-2-oxo-3-pyrrolinyl-1-carboxamido)-ethyl]-phenylsulfonyl-}3-ethylharnstoff,
(6) 1-{2-Ethoxy-5-[2-(3-ethyl-4-methyl-2-oxo-3-pyrrolinyl-1-carboxamido)-ethyl]-phenylsulfonyl-}3-(1-propyl)-harnstoff,
(7) 1-{2-Ethoxy-5-[2-(3-ethyl-4-methyl-2-oxo-3-pyrrolinyl-1-carboxamido)-ethyl]-phenylsulfonyl-}3-(2-propyl)-harnstoff,
(8) 1-{2-Ethoxy-5-[2-(3-ethyl-4-methyl-2-oxo-3-pyrrolinyl-1-carboxamido)-ethyl]-phenylsulfonyl-}3-(cyclopropyl)-harnstoff,
(9) 1-{2-(2,2,2-Trifluorethoxy)-5-[2-(3-ethyl-4-methyl-2-oxo-3-pyrrolinyl-1-carboxamido)-ethyl]-phenylsulfonyl-}3-ethylharnstoff,
(10) 1-{2-(2,2,2-Trifluorethoxy)-5-[2-(3-ethyl-4-methyl-2-oxo-3-pyrrolinyl-1-carboxamido)-ethyl]-phenylsulfonyl-}3-(1-propyl)-harnstoff,
(11) 1-{2-(2,2,2-Trifluorethoxy)-5-[2-(3-ethyl-4-methyl-2-oxo-3-pyrrolinyl-1-carboxamido)-ethyl]-phenylsulfonyl-}3-(2-propyl)-harnstoff,
(12) 1-{2-(2,2,2-Trifluorethoxy)-5-[2-(3-ethyl-4-methyl-2-oxo-3-pyrrolinyl-1-carboxamido)-ethyl]-phenylsulfonyl-}3-(cyclopropyl)-harnstoff,
(13) 1-{2-Thiomethyl-5-[2-(3-ethyl-4-methyl-2-oxo-3-pyrrolinyl-1-carboxamido)-ethyl]-phenylsulfonyl-}3-ethylharnstoff,
(14) 1-{2-Thiomethyl-5-[2-(3-ethyl-4-methyl-2-oxo-3-pyrrolinyl-1-carboxamido)-ethyl]-phenylsulfonyl-}3-(1-propyl)-harnstoff,
(15) 1-{2-Thiomethyl-5-[2-(3-ethyl-4-methyl-2-oxo-3-pyrrolinyl-1-carboxamido)-ethyl]-phenylsulfonyl-}3-(2-propyl)-harnstoff,
(16) 1-{2-Thiomethyl-5-[2-(3-ethyl-4-methyl-2-oxo-3-pyrrolinyl-1-carboxamido)-ethyl]-phenylsulfonyl-}3-(cyclopropyl)-harnstoff,
(17) 1-{2-(N,N-Dimethylamino)-5-[2-(3-ethyl-4-methyl-2-oxo-3-pyrrolinyl-1-carboxamido)-ethyl]-phenylsulfonyl-}3-ethylharnstoff,
(18) 1-{2-(N,N-Dimethylamino)-5-[2-(3-ethyl-4-methyl-2-oxo-3-pyrrolinyl-1-carboxamido)-ethyl]-phenylsulfonyl-}3-(1-propyl)-harnstoff,
(19) 1-{2-(N,N-Dimethylamino)-5-[2-(3-ethyl-4-methyl-2-oxo-3-pyrrolinyl-1-carboxamido)-ethyl]-phenylsulfonyl-}3-(2-propyl)-harnstoff,
(20) 1-{2-(N,N-Dimethylamino)-5-[2-(3-ethyl-4-methyl-2-oxo-3-pyrrolinyl-1-carboxamido)-ethyl]-phenylsulfonyl-}3-(cyclopropyl)-harnstoff,
(21) 1-{2-(4-Morpholino)-5-[2-(3-ethyl-4-methyl-2-oxo-3-pyrrolinyl-1-carboxamido)-ethyl]-phenylsulfonyl-}3-ethylharnstoff,
(22) 1-{2-(4-Morpholino)-5-[2-(3-ethyl-4-methyl-2-oxo-3-pyrrolinyl-1-carboxamido)-ethyl]-phenylsulfonyl-}3-(1-propyl)-harnstoff,
(23) 1-{2-(4-Morpholino)-5-[2-(3-ethyl-4-methyl-2-oxo-3-pyrrolinyl-1-carboxamido)-ethyl]-phenylsulfonyl-}3-(2-propyl)-harnstoff,
(24) 1-{2-(4-Morpholino)-5-[2-(3-ethyl-4-methyl-2-oxo-3-pyrrolinyl-1-carboxamido)-ethyl]-phenylsulfonyl-}3-(cyclopropyl)-harnstoff,
(25) 1-{2-Methyl-5-[2-(3-ethyl-4-methyl-2-oxo-3-pyrrolinyl-1-carboxamido)-ethyl]-phenylsulfonyl-}3-ethylharnstoff,
(26) 1-{2-Methyl-5-[2-(3-ethyl-4-methyl-2-oxo-3-pyrrolinyl-1-carboxamido)-ethyl]-phenylsulfonyl-}3-(1-propyl)-harnstoff,
(27) 1-{2-Methyl-5-[2-(3-ethyl-4-methyl-2-oxo-3-pyrrolinyl-1-carboxamido)-ethyl]-phenylsulfonyl-}3-(2-propyl)-harnstoff,
(28) 1-{2-Methyl-5-[2-(3-ethyl-4-methyl-2-oxo-3-pyrrolinyl-1-carboxamido)-ethyl]-phenylsulfonyl-}3-(cyclopropyl)-harnstoff,
(29) 1-{2-Fluor-5-[2-(3-ethyl-4-methyl-2-oxo-3-pyrrolinyl-1-carboxamido)-ethyl]-phenylsulfonyl-}3-ethylharnstoff,
(30) 1-{2-Fluor-5-[2-(3-ethyl-4-methyl-2-oxo-3-pyrrolinyl-1-carboxamido)-ethyl]-phenylsulfonyl-}3-(1-propyl)-harnstoff,
(31) 1-{2-Fluor-5-[2-(3-ethyl-4-methyl-2-oxo-3-pyrrolinyl-1-carboxamido)-ethyl]-phenylsulfonyl-}3-(2-propyl)-harnstoff,
(32) 1-{2-Fluor-5-[2-(3-ethyl-4-methyl-2-oxo-3-pyrrolinyl-1-carboxamido)-ethyl]-phenylsulfonyl-}3-(cyclopropyl)-harnstoff,
(33) 1-{2-Methoxy-5-[2-(3-ethyl-4-methyl-2-oxo-3-pyrrolinyl-1-carboxamido)-ethyl]-phenylsulfonyl-}3-ethylthioharnstoff,
(34) 1-{2-Methoxy-5-[2-(3-ethyl-4-methyl-2-oxo-3-pyrrolinyl-1-carboxamido)-ethyl]-phenylsulfonyl-}3-(1-propyl)-thioharnstoff
(35) 1-{2-Methoxy-5-[2-(3-ethyl-4-methyl-2-oxo-3-pyrrolinyl-1-carboxamido)-ethyl]-phenylsulfonyl-}3-(1-propyl)-thioharnstoff,
(36) 1-{2-Methoxy-5-[2-(3-ethyl-4-methyl-2-oxo-3-pyrrolinyl-1-carboxamido)-ethyl]-phenylsulfonyl-}3-(2-propyl)-thioharnstoff,
(37) 1-{2-Methoxy-5-[2-(3-ethyl-4-methyl-2-oxo-3-pyrrolinyl-1-carboxamido)-ethyl]-phenylsulfonyl-}3-(cyclopropyl)-thioharnstoff,
(38) 1-{2-Ethoxy-5-[2-(3-ethyl-4-methyl-2-oxo-3-pyrrolinyl-1-carboxamido)-ethyl]-phenylsulfonyl-}3-ethylthioharnstoff,
(39) 1-{2-Ethoxy-5-[2-(3-ethyl-4-methyl-2-oxo-3-pyrrolinyl-1-carboxamido)-ethyl]-phenylsulfonyl-}3-(1-propyl)-thioharnstoff,
(40) 1-{2-Ethoxy-5-[2-(3-ethyl-4-methyl-2-oxo-3-pyrrolinyl-1-carboxamido)-ethyl]-phenylsulfonyl-}3-(2-propyl)-thioharnstoff
(41) 1-{2-Ethoxy-5-[2-(3-ethyl-4-methyl-2-oxo-3-pyrrolinyl-1-carboxamido)-ethyl]-phenylsulfonyl-}3-(cyclopropyl)-thioharnstoff,
(42) 1-{2-(2,2,2-Trifluorethoxy)-5-[2-(3-ethyl-4-methyl-2-oxo-3-pyrrolinyl-1-carboxamido)-ethyl]-phenylsulfonyl-}3-ethylthioharnstoff,
(43) 1-{2-(2,2,2-Trifluorethoxy)-5-[2-(3-ethyl-4-methyl-2-oxo-3-pyrrolinyl-1-carboxamido)-ethyl]-phenylsulfonyl-}3-(1-propyl)-thioharnstoff,
(44) 1-{2-(2,2,2-Trifluorethoxy)-5-[2-(3-ethyl-4-methyl-2-oxo-3-pyrrolinyl-1-carboxamido)-ethyl]-phenylsulfonyl-}3-(2-propyl)-thioharnstoff,
(45) 1-{2-(2,2,2-Trifluorethoxy)-5-[2-(3-ethyl-4-methyl-2-oxo-3-pyrrolinyl-1-carboxamido)-ethyl]-phenylsulfonyl-}3-(cyclopropyl)-thioharnstoff,
(46) 1-{2-Thiomethyl-5-[2-(3-ethyl-4-methyl-2-oxo-3-pyrrolinyl-1-carboxamido)-ethyl]-phenylsulfonyl-}3-ethylthioharnstoff,
(47) 1-{2-Thiomethyl-5-[2-(3-ethyl-4-methyl-2-oxo-3-pyrrolinyl-1-carboxamido)-ethyl]-phenylsulfonyl-}3-(1-propyl)-thioharnstoff,
(48) 1-{2-Thiomethyl-5-[2-(3-ethyl-4-methyl-2-oxo-3-pyrrolinyl-1-carboxamido)-ethyl]-phenylsulfonyl-}3-(2-propyl)-thioharnstoff,
(49) 1-{2-Thiomethyl-5-[2-(3-ethyl-4-methyl-2-oxo-3-pyrrolinyl-1-carboxamido)-ethyl]-phenylsulfonyl-}3-(cyclopropyl)-thioharnstoff,
(50) 1-{2-(N,N-Dimethylamino)-5-[2-(3-ethyl-4-methyl-2-oxo-3-pyrrolinyl-1-carboxamido)-ethyl]-phenylsulfonyl-}3-ethylthioharnstoff,
(51) 1-{2-(N,N-Dimethylamino)-5-[2-(3-ethyl-4-methyl-2-oxo-3-pyrrolinyl-1-carboxamido)-ethyl]-phenylsulfonyl-}3-(1-propyl)-thioharnstoff,
(52) 1-{2-(N,N-Dimethylamino)-5-[2-(3-ethyl-4-methyl-2-oxo-3-pyrrolinyl-1-carboxamido)-ethyl]-phenylsulfonyl-}3-(2-propyl)-thioharnstoff,
(53) 1-{2-(N,N-Dimethylamino)-5-[2-(3-ethyl-4-methyl-2-oxo-3-pyrrolinyl-1-carboxamido)-ethyl]-phenylsulfonyl-}3-(cyclopropyl)-thioharnstoff,
(54) 1-{2-(4-Morpholino)-5-[2-(3-ethyl-4-methyl-2-oxo-3-pyrrolinyl-1-carboxamdo)-ethyl]-phenylsulfonyl-}3-ethylthioharnstoff,
(55) 1-{2-(4-Morpholino)-5-[2-(3-ethyl-4-methyl-2-oxo-3-pyrrolinyl-1-carboxamido)-ethyl]-phenylsulfonyl-}3-(1-propyl)-thioharnstoff,
(56) 1-{2-(4-Morpholino)-5-[2-(3-ethyl-4-methyl-2-oxo-3-pyrrolinyl-1-carboxamido)-ethyl]-phenylsulfonyl-}3-(2-propyl)-thioharnstoff,
(57) 1-{2-(4-Morpholino)-5-[2-(3-ethyl-4-methyl-2-oxo-3-pyrrolinyl-1-carboxamdo)-ethyl]-phenylsulfonyl-}3-(cyclopropyl)-thioharnstoff,
(58) 1-{2-Methyl-5-[2-(3-ethyl-4-methyl-2-oxo-3-pyrrolinyl-1-carboxamido)-ethyl]-phenylsulfonyl-}3-ethylthioharnstoff,
(59) 1-{2-Methyl-5-[2-(3-ethyl-4-methyl-2-oxo-3-pyrrolinyl-1-carboxamido)-ethyl]-phenylsulfonyl-}3-(1-propyl)-thioharnstoff,
(60) 1-{2-Methyl-5-[2-(3-ethyl-4-methyl-2-oxo-3-pyrrolinyl-1-carboxamido)-ethyl]-phenylsulfonyl-}3-(2-propyl)-thioharnstoff,
(61) 1-{2-Methyl-5-[2-(3-ethyl-4-methyl-2-oxo-3-pyrrolinyl-1-carboxamido)-ethyl]-phenylsulfonyl-}3-(cyclopropyl)-thioharnstoff,
(62) 1-{2-Fluor-5-[2-(3-ethyl-4-methyl-2-oxo-3-pyrrolinyl-1-carboxamido)-ethyl]-phenylsulfonyl-}3-ethylthioharnstoff,
(63) 1-{2-Fluor-5-[2-(3-ethyl-4-methyl-2-oxo-3-pyrrolinyl-1-carboxamido)-ethyl]-phenylsulfonyl-}3-(1-propyl)-thioharnstoff,
(64) 1-{2-Fluor-5-[2-(3-ethyl-4-methyl-2-oxo-3-pyrrolinyl-1-carboxamido)-ethyl]-phenylsulfonyl-}3-(2-propyl)-thioharnstoff,
(65) 1-{2-Fluor-5-[2-(3-ethyl-4-methyl-2-oxo-3-pyrrolinyl-1-carboxamido)-ethyl]-phenylsulfonyl-}3-(cyclopropyl)-thioharnstoff.

### Beispiel 1:

### 1-{2-Methoxy-5-[2-(3-ethyl-4-methyl-2-oxo-3-pyrrolinyl-1-carboxamido)-ethyl]-phenylsulfonyl-}3-methylthioharnstoff

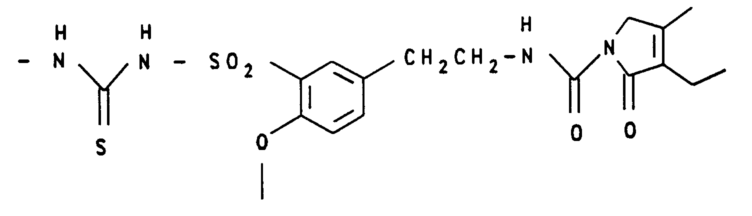

0.22 g (0.58 mmol) 2-Methoxy-5-[2-(3-ethyl-4-methyl-2-oxo-3-pyrrolinyl-1-carboxamido)-ethyl]-benzolsulfonamid werden in 10 ml Aceton gelöst und mit 25 mg NaOH und 50 µl Wasser versetzt. Unter Eiskühlung gibt man 45 mg (0.6 mmol) Methylisothiocyanat in die gut gerührte Reaktionsmischung, rührt eine Stunde bei Raumtemperatur nach und entfernt anschließend das Lösungsmittel bei vermindertem Druck. Der verbleibende Rückstand wird an Kieselgel 60 säulenchromatographisch unter Zuhilfenahme des Laufmittels Ethylacetat aufgereinigt (R_{F} = 0.4) und liefert 0.18 g des gewünschten Produkts als farblose Kristalle, die bei 65 bis 70°C schmelzen.

### Darstellung der Ausgangsverbindung

4.53 g (30 mmol) 4-Methoxy-β-phenylethylamin werden tropfenweise zu einer Lösung von 2.96 g (10.0 mmol) Triphosgen in 30 ml trockenem Tetrahydrofuran gegeben und 2 Stunden zum Sieden erhitzt. Die auf Raumtemperatur abgekühlte Lösung wird filtriert und das Filtrat im Vakuum eingeengt. Das verbleibende farblose Öl wird durch Kugelrohr-Destillation bei einem Druck von ca. 2.7 hPa (2 Torr) und einer Badtemperatur von 220°C gereinigt und liefert 1.1 g 4-Methoxy-β-phenylethylisocyanat.
0.7 g (4.0 mmol) 4-Methoxy-β-phenylethylisocyanat und 0.4 g (3.5 mmol) 3-Ethyl-4-methyl-2-oxo-3-pyrrolin werden miteinander verrührt und eine Stunde lang auf 150°C erhitzt. Die abgekühlte Reaktionsmischung wird in einem Minimum Ethylacetat gelöst und an Kieselgel 60 säulenchromatographisch unter Zuhilfenahme des Laufmittels Ethylacetat aufgereinigt (R_{F} = 0.5). Man erhält nach Abdampfen des Eluens bei vermindertem Druck 0.25 g Methoxy-4-[2-(3-ethyl-4-methyl-2-oxo-3-pyrrolinyl-1-carboxamido)-ethyl]-benzol, das portionsweise in 0°C kalte Chlorsulfonsäure eingetragen wird. Diese Reaktionsmischung wird nach einer Stunde Rühren bei 0°C auf Eis gegossen. Das ausgefallene Sulfonsäurechlorid wird in Ethylacetat aufgenommen. Die organische Phase wird mit Natriumsulfat getrocknet, im Vakuum eingeengt und liefert das Sulfonsäurechlorid als Rohprodukt, das in Aceton gelöst und mit wäßriger Ammoniaklösung in 2-Methoxy-5-[2-(3-ethyl-4-methyl-2-oxo-3-pyrrolinyl-1-carboxamido)-ethyl]-benzolsulfonamid überführt wird. Das Sulfonamid schmilzt bei 204 - 205°C.

### Beispiel 2

### 1-{2-Methoxy-5-[2-(3-ethyl-4-methyl-2-oxo-3-pyrrolinyl-1-carboxamido)-ethyl]-phenylsulfonyl-}3-methylharnstoff

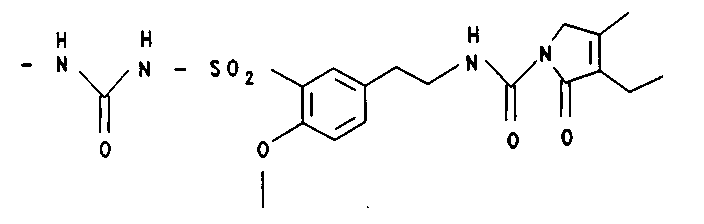

0.25 g (0.55 mmol) 1-{2-Methoxy-5-[2-(3-ethyl-4-methyl-2-oxo-3-pyrrolinyl-1-carboxamido)-ethyl]-phenylsulfonyl-}3-methylthioharnstoff werden in wäßriger Natronlauge (45 mg NaOH und 0.5 ml Wasser) suspendiert und bei 40°C mit 35%igem, wäßrigem Wasserstoffperoxid versetzt und 30 min bei 80°C nachgerührt. Die Reaktionsmischung wird bei Raumtemperatur mit Dichlormethan versetzt und mit etwas 2 normaler, wäßriger Salzsäure angesäuert (pH = 2 - 3) und mit Natriumsulfat getrocknet. Die organische Phase wird im Vakuum eingeengt und liefert 0.22 g 1-{2-Methoxy-5-[2-(3-ethyl-4-methyl-2-oxo-3-pyrrolinyl-1-carboxamido)-ethyl]-phenylsulfonyl-}3-methylharnstoff als farblose Kristalle vom Schmelzpunkt 184 - 187°C.

### Beispiel 3

### 1-{2-Fluor-5-[2-(3-ethyl-4-methyl-2-oxo-3-pyrrolinyl-1-carboxamido)-ethyl]-phenylsulfonyl-}3-methylthioharnstoff

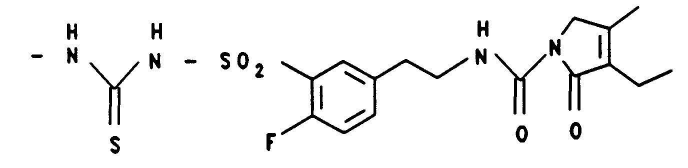

In Anlehnung an Beispiel 1 läßt sich 1-{2-Fluor-5-[2-(3-ethyl-4-methyl-2-oxo-3-pyrrolinyl-1-carboxamido)-ethyl]-phenylsulfonyl-}3-methylthioharnstoff (Schmp.: 90°C unter Zersetzung) ausgehend von 4-Fluor-β-phenylethylamin via Fluor-4-[2-(3-ethyl-4-methyl-2-oxo-3-pyrrolinyl-1-carboxamido)-ethyl]-benzol (Schmp.: 141°C), 2-Fluor-5-[2-(3-ethyl-4-methyl-2-oxo-3-pyrrolinyl-1-carboxamido)-ethyl]-benzolsulfonsäurechlorid (Schmp.: 147°C), 2-Fluor-5-[2-(3-ethyl-4-methyl-2-oxo-3-pyrrolinyl-1-carboxamido)-ethyl]-benzolsulfonamid (Schmp.: 136°C) synthetisieren.

### Beispiel 4

### 1-[2-Ethoxy-5-[2-(3-ethyl-4-methyl-2-oxo-3-pyrrolinyl-1-carboxamido)-ethyl]-phenylsulfonyl]-3-methylthioharnstoff

Schmp.: 176 - 178°C
analog Beispiel 1

### Beispiel 5

### 1-[2-Ethoxy-5-[2-(3-ethyl-4-methyl-2-oxo-3-pyrrolinyl-1-carboxamido)-ethyl]-phenylsulfonyl]-3-methylharnstoff

Schmp.: 160 - 165°C
analog Beispiel 2

### Pharmakologische Daten:

Mit den folgenden Modellen können die therapeutischen Eigenschaften der Verbindungen I nahegelegt werden:

### (1) Aktionspotentialdauer am Papillarmuskel des Meerschweinchens:

### (a) Einleitung

ATP-Mangelzustände, wie sie während einer Ischämie in der Herzmuskelzelle beobachtet werden, führen zu einer Verkürzung der Aktionspotentialdauer. Sie gelten als einer der Ursachen für sogenannte Reentryarrhythmien, die den plötzlichen Herztod verursachen können. Die Öffnung von ATP-sensitiven K-Kanälen durch das Absinken von ATP gilt hierfür als ursächlich.

### (b) Methode

Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9 % NaCl, 0.048 % KCl, 0.024 % CaCl₂, 0.02 % NaHCO₃, und 0.1 % Glucose) durchspült und mit einer Mischung aus 95 % Sauerstoff und 5 % Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2 x 10⁻⁵ Mol pro Liter zugesetzt. Das Aktionspotential wird mit einem Amplifier von Hugo Sachs verstärkt und am Oszilloskop dargestellt. Die Dauer des Aktionspotentials wird bei einem Repolarisierungsgrad von 95 % (APD₉₅) bestimmt. Aktionspotentialverkürzungen werden entweder durch Zugabe einer 1µM starken Lösung des Kaliumkanalöffners Rilmakalim (Hoe 234) [W. Linz, E. Klaus, U. Albus, R.H.A. Becker, D. Mania, H.C. Englert, B.A. Schölkens Arznemittelforschung/Drug Research, Band 42 (II), 1992, S. 1180 - 1185] oder durch Zugabe von 2-Desoxyglucose (DEO) hervorgerufen. Durch 2-Desoxyglucose werden in der experimentellen Physiologie ATP-Mangelzustände durch Blockade des Glucosestoffwechsels hervorgerufen. Der aktionspotential-verkürzende Effekt dieser Substanzen wurde durch die gleichzeitige Gabe der Testsubstanzen verhindert oder verringert. Testsubstanzen wurden als Stammlösungen in Propandiol der Badlösung zugesetzt. Die angegebenen Werte beziehen sich auf Messungen 30 min nach der Zugabe. Als Kontrolle gilt die APD₉₅ in Gegenwart von DEO oder Hoe 234 und in Abwesenheit der Testsubstanz.

### (c) Resultate:

Folgende Werte wurden gemessen:

| Messung | APD₉₅-DEO^{a)} [ms] | APD₉₅-HOE 234^{a)} [ms] |
|---|---|---|
| Kontrolle | < 40 | < 40 |
| Beispiel 1 | 60 ± 22 (162 ± 7) n = 3 | 119 ± 16 (162 ± 16) n = 3 |
| Beispiel 2 | 110 (155) n = 1 | 125 (185) n = 1 |

| | | |
|---|---|---|
| a) den Meßwerten aus n-Versuchen sind die korrespondierenden Leerwerte in Klammern nachgestellt. Die Leerwerte sind die APD₉₅-Werte zu Versuchsbeginn ohne DEO, Hoe 234 und Testsubstanz in der Ringerlösung. | | |

### (2) Membranpotential an isolierten β-Zellen:

### (a) Einleitung

Der Wirkmechanismus der blutzuckersenkenden Sulfonylharnstoffe ist in groben Zügen aufgeklärt. Zielorgan sind die β-Zellen des Pankreas wo es zu einer Mehrausschüttung des blutzuckersenkenden Hormons Insulin kommt. Die Freisetzung von Insulin wird über das Zellmembranpotential gesteuert. Glibenclamid bewirkt eine Depolarisation der Zellmembran, was über einen vermehrten Einstrom von Calciumionen die Insulinfreisetzung fördert. Das Ausmaß dieser Depolarisation der Zellmembran ΔU wurde an RINm5F Zellen, einer Pankreastumorzellinie, für einige der erfindungsgemäßen Verbindungen bestimmt. Die Wirkstärke einer Verbindung in diesem Modell sagt das Ausmaß des blutzuckersenkenden Potentials dieser Verbindung voraus.

### (b) Methode

### Zellkultur von RINm5F Zellen

RINm5F Zellen wurden in RPMI 1640 Kulturmedium (Flow), dem 11 mmol Glukose, 10 % (vol/vol) fötales Kälberserum, 2 mmol Glutamin und 50 µg/ml Gentamycin zugesetzt wurde, bei 37°C kultiviert. Für die Untersuchungen wurden die Zellen durch Inkubation (ca. 3 min) in einem Ca²⁺-freien Medium, das 0.25 % Trypsin enthielt, isoliert und auf Eis aufbewahrt.

### Meßmethode

Isolierte RINm5F Zellen wurden in eine Plexiglaskammer auf einem inversen Mikroskop, das mit Differential-Interferenz-Kontrast Optik ausgerüstet ist, gebracht. Unter optischer Kontrolle (400-fache Vergrößerung) wurde eine feuerpolierte Mikropipette mit Öffnungsdurchmessser von etwa 1 µm mit Hilfe eines Mikromanipulators auf die Zelle aufgesetzt. Durch Anlegen eines leichten Unterdruckes in der Patch-Pipette wurde zunächst eine hohe elektrische Abdichtung zwischen Glas und Zellmembran hergestellt und anschließend durch Erhöhung des Unterdruckes der Membranfleck unter der Meßpipette aufgerissen. In dieser Ganzzellenkonfiguration wurde mit Hilfe eines Patch-Clamp Verstärkers (L/M EPC 7) das Zellpotential registriert und durch Anlegen einer Spannungsrampe der Ganzzellenstrom gemessen.

Lösungen: Die Patch-Pipette war mit KCl-Lösung gefüllt (in mmol): 140 KCl, 10 NaCl, 1.1 MgCl₂, 0.5 EGTA, 1 Mg-ATP, 10 HEPES, pH = 7.2 und im Bad befand sich NaCl-Lösung (in mmol): 140 NaCl, 4.7 KCl, 1.1 MgCl₂, 2 CaCl₂, 10 HEPES, pH = 7.4. Von den Testsubstanzen wurden Stocklösungen (Konzentration 100 mmol) in Dimethylsulfoxid (DMSO) und entsprechende Verdünnungen in NaCl-Lösung hergestellt. DMSO alleine hatte keinen Effekt auf das Zellpotential. Um das Zellpotential unter Kontrollbedingungen zu stabilisieren, wurde der Öffner für ATP-sensitive K⁺-Kanäle Diazoxid (100 µmol) bei allen Versuchen in die Badlösung zugegeben. Alle Experimente wurden bei 34 ± 1°C durchgeführt.

### (c) Ergebnisse (Die Konzentrationen der erfindungsgemäßen Verbindungen betragen in den Versuchen 10⁻⁵ Mol pro Liter)

| Messung | ΔU (mV)^{a)} |
|---|---|
| Beispiel 1 | 2 (-80) n = 6 |
| Beispiel 2 | 12 (- 71) n = 6 |

| | |
|---|---|
| ^{a}) den Meßwerten aus n-Versuchen sind die korrespondierenden Leerwerte in Klammern nachgestellt. Die Leerwerte sind die Zellpotentiale unter Diazoxid-Gabe. | |

## Patentansprüche

1. Substituierte Benzolsulfonylharnstoffe und -thioharnstoffe der Formel I, 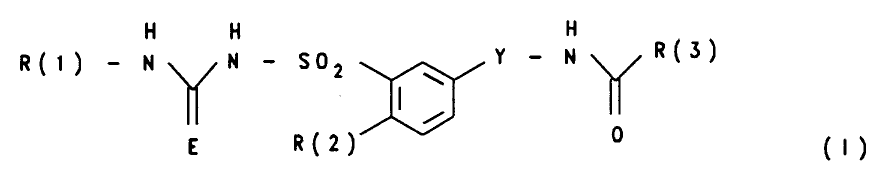 worin bedeuten:
R(1) (C₁-C₆)-Alkyl, (C₃-C₆)-Cycloalkyl, CₐH₂ₐ-(C₃-C₅)-Cycloalkyl oder C_{c}H_{2c}-C_{d}F_{2d+1};
a 1, 2 oder 3;
c 0, 1, 2 oder 3;
d 1, 2, 3, 4, 5 oder 6;
R(2) F, Cl, Br, I, (C₁-C₆)-Alkyl, (C₁-C₆)-Alkoxy, (C₁-C₆)-Mercaptoalkyl, (C₁-C₆)-Fluoralkoxy, (C₁-C₆)-Fluoralkyl, (C₃-C₆)-Cycloalkyl oder NR(4)R(5);
R(4) und R(5) gemeinsam eine (CH₂)₄₋₆-Kette, in der eine der CH₂-Gruppen durch Sauerstoff, Schwefel oder NR(6) ersetzt sein kann, wobei zwischen dem N-Atom des NR(4)R(5) und dem Sauerstoff, Schwefel oder NR(6) mindestens eine CH₂-Gruppe stehen muß;
oder
R(4), R(5), R(6) unabhängig voneinander Wasserstoff, (C₁-C₆)-Alkyl, (C₃-C₆)-Cycloalkyl oder (C₃-C₆)-Alkenyl;
E Sauerstoff oder Schwefel;
Y eine Kohlenwasserstoffkette -[CR(7)₂]ₙ-;
R(7) H oder (C₁-C₂)-Alkyl;
n 1, 2, 3 oder 4;
R(3) einen Heterocyclus der Formel 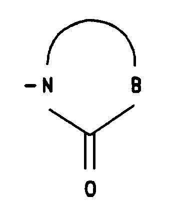
B (C₃-C₆)-Alkenylen, das unsubstituiert oder mit bis zu 3 (C₁-C₄)-Alkylgruppen oder mit einem Phenylrest substituiert ist;
und ihre physiologisch unbedenklichen Salze.

2. Verbindungen der Formel I nach Anspruch 1, in welcher bedeuten:
R(1) (C₁-C₄)-Alkyl, (C₃-C₅)-Cycloalkyl oder C_{c}H_{2c}-C_{d}F_{2d+1};
c 0, 1, 2 oder 3;
d 1, 2, 3, 4, 5 oder 6;
R(2) F, Cl, (C₁-C₆)-Alkyl, (C₁-C₆)-Alkoxy, (C₁-C₆)-Mercaptoalkyl, (C₁-C₆)-Fluoralkoxy, (C₁-C₆)-Fluoralkyl, (C₃-C₆)-Cycloalkyl oder NR(4)R(5);
R(4) und R(5) gemeinsam eine (CH₂)₄₋₆-Kette, in der eine der CH₂-Gruppen durch Sauerstoff, Schwefel oder NR(6) ersetzt sein kann, wobei zwischen dem N-Atom des NR(4)R(5) und dem Sauerstoff, Schwefel oder NR(6) mindestens eine CH₂-Gruppe stehen muß;
oder
R(4), R(5), R(6) unabhängig voneinander Wasserstoff, (C₁-C₆)-Alkyl, (C₃-C₆)-Cycloalkyl oder (C₃-C₆)-Alkenyl;
und ihre physiologisch unbedenklichen Salze.

3. Verbindungen der Formel I nach Anspruch 1 und/oder Anspruch 2, in welcher bedeuten:
R(1) (C₁-C₄)-Alkyl, (C₃-C₄)-Cycloalkyl oder (C₁-C₄)-Fluoralkyl;
R(2) F, Cl, (C₁-C₄)-Alkyl, (C₁-C₄)-Fluoralkyl oder (C₃-C₆)-Cycloalkyl;
Y eine Kohlenwasserstoffkette -[CR(7)₂]ₙ-;
R(7) H oder (C₁-C₂)-Alkyl;
n 2 oder 3;
und ihre physiologisch unbedenklichen Salze.

4. Verbindungen der Formel I nach Anspruch 1 und/oder Anspruch 2, in welcher bedeuten:
R(1) (C₁-C₄)-Alkyl, (C₃-C₄)-Cycloalkyl oder (C₁-C₄)-Fluoralkyl;
R(2) (C₁-C₄)-Alkoxy, (C₁-C₄)-Mercaptoalkyl oder (C₁-C₄)-Fluoralkoxy;
Y eine Kohlenwasserstoffkette -[CR(7)₂]ₙ-;
R(7) H oder (C₁-C₂)-Alkyl;
n 2 oder 3;
R(3) einen Heterocyclus der Formel 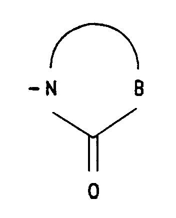
B (C₃-C₄)-Alkenylen, das unsubstituiert oder mit ein bis drei (C₁-C₄)-Alkylgruppen oder mit einem Phenylrest substituiert ist;
und ihre physiologisch unbedenklichen Salze.

5. Verbindungen der Formel I nach Anspruch 1 und/oder Anspruch 2, in welcher bedeuten:
R(1) (C₁-C₄)-Alkyl oder (C₁-C₄)-Fluoralkyl;
R(2) NR(4)R(5);
R(4) und R(5) gemeinsam eine (CH₂)₄₋₆-Kette, in der eine der CH₂-Gruppen durch Sauerstoff, Schwefel oder NR(6) ersetzt sein kann, wobei zwischen dem N-Atom des NR(4)R(5) und dem Sauerstoff, Schwefel oder NR(6) mindestens eine CH₂-Gruppe stehen muß;
oder
R(4), R(5) unabhängig voneinander CH₃, C₂H₅, n-C₃H₇, Iso-C₃H, oder Cyclo-C₃H₅;
R(6) H, CH₃ oder C₂H₅;
Y eine Kohlenwasserstoffkette -[CR(7)₂]ₙ-;
R(7) H oder (C₁-C₂)-Alkyl;
n 2 oder 3;
R(3) einen Heterocyclus der Formel 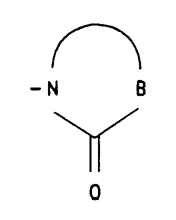
B (C₃-C₄)-Alkenylen, das unsubstituiert oder mit bis zu 3 (C₁-C₄)-Alkylgruppen oder mit einem Phenylrest substituiert ist;
und ihre physiologisch unbedenklichen Salze.

6. Verfahren zur Herstellung einer Verbindung der Formel I nach einem oder mehreren der Ansprüche 1 bis 5, dadurch gekennzeichnet, daß man
(a) ein Sulfonamid der Formel II oder dessen Salz der Formel III, 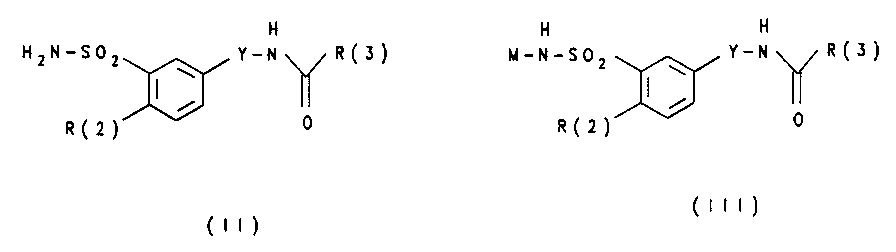 worin M das Kation eines Alkali- oder Erdalkalimetalls ist, mit einem R(1)-substituierten Isocyanat der Formel IV
R(1) - N = C = O (IV)
zu einem substituierten Benzolsulfonylharnstoff der Formel I a umsetzt, 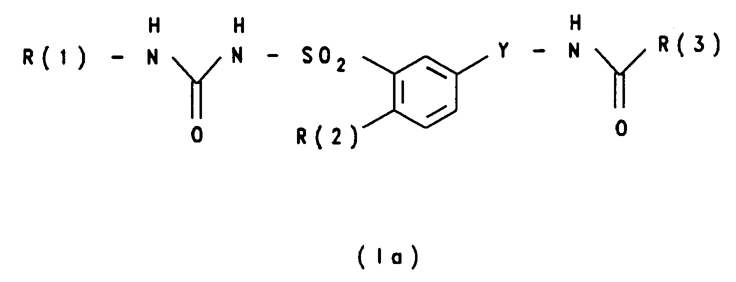 oder daß man
(b) einen Benzolsulfonylharnstoff der Formel I a aus einem aromatischen Benzolsulfonamid der Formel II oder dessen Salz der Formel III und einem R(1)-substituierten Trichloracetamid der Formel V 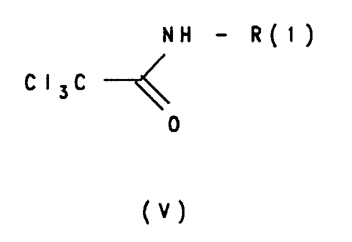 herstellt,
oder daß man
(c) einen Benzolsulfonylthioharnstoff der Formel I b 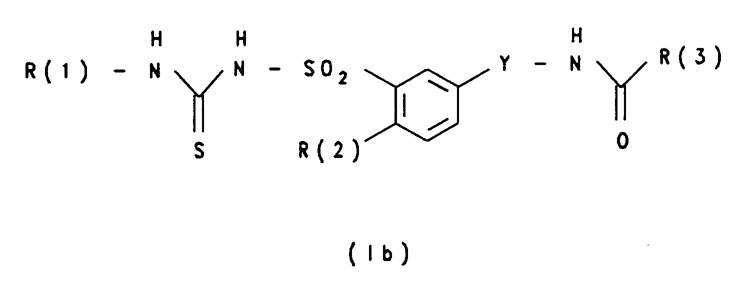 aus einem Benzolsulfonamid der Formel II oder dessen Salz der Formel III und einem R(1)-substituierten Thioisocyanat der Formel VI
R(1) - N = C = S (VI)
herstellt,
oder daß man
(d) einen substituierten Benzolsulfonylharnstoff der Formel I a durch eine Umwandlungsreaktion aus einem Benzolsulfonylthioharnstoff der Formel I b herstellt,
oder daß man
(e) einen Benzolsulfonylharnstoff der Formel I a durch Umsetzung eines Amins der Formel R(1)-NH₂ mit einem Benzolsulfonylisocyanat der Formel VII 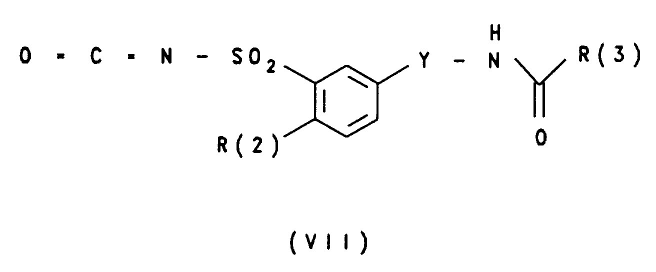 herstellt,
oder daß man
(f) einen Benzolsulfonylthioharnstoff der Formel I b durch Umsetzung eines Amins der Formel R(1)-NH₂ mit einem Benzolsulfonylisothiocyanat der Formel VIII 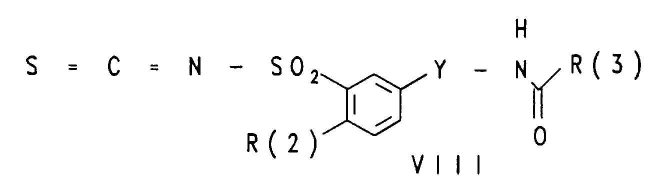 herstellt.

7. Verwendung einer Verbindung der Formel I nach einem oder mehreren der Ansprüche 1 bis 5 und/oder eines physiologisch unbedenklichen Salzes davon zur Herstellung eines Medikaments zur Behandlung von Herzrhythmusstörungen.

8. Verwendung einer Verbindung der Formel I nach einem oder mehreren der Ansprüche 1 bis 5 und/oder eines physiologisch unbedenklichen Salzes davon zur Herstellung eines Medikaments zur Verhinderung des plötzlichen Herztods.

9. Verwendung einer Verbindung der Formel I nach einem oder mehreren der Ansprüche 1 bis 5 und/oder eines physiologisch unbedenklichen Salzes davon zur Herstellung eines Medikaments zur Behandlung von ischämischen Zuständen des Herzens.

10. Verwendung einer Verbindung der Formel I nach einem oder mehreren der Ansprüche 1 bis 5 und/oder eines physiologisch unbedenklichen Salzes davon zur Herstellung eines Medikaments zur Behandlung der geschwächten Herzkraft.

11. Verwendung einer Verbindung der Formel I nach einem oder mehreren der Ansprüche 1 bis 5 und/oder eines physiologisch unbedenklichen Salzes davon zur Herstellung eines Medikaments zur Verbesserung der Herzfunktion nach Herztransplantation.

12. Heilmittel, gekennzeichnet durch eine wirksame Menge an einer Verbindung der Formel I nach einem oder mehreren der Ansprüche 1 bis 5 und/oder eines physiologisch unbedenklichen Salzes davon.

## Claims

1. A substituted benzenesulfonylurea or -thiourea of the formula I 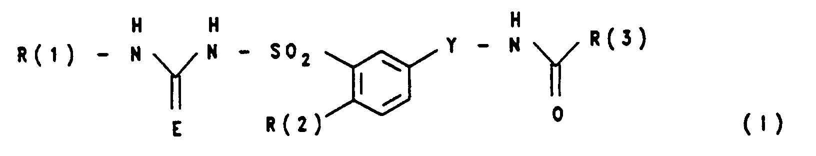 in which:
R(1) is (C₁-C₆)-alkyl, (C₃-C₆)-cycloalkyl, CₐH₂ₐ-(C₃-C₅)-cycloalkyl, or C_{c}H_{2c}-C_{d}F_{2d+1};
a is 1, 2 or 3;
c is 0, 1, 2 or 3;
d is 1, 2, 3, 4, 5 or 6;
R(2) is F, Cl, Br, I, (C₁-C₆)-alkyl, (C₁-C₆)-alkoxy, (C₁-C₆)-mercaptoalkyl, (C₁-C₆)-fluoroalkoxy, (C₁-C₆)-fluoroalkyl, (C₃-C₆)-cycloalkyl or NR(4)R(5);
R(4) and R(5) together are a (CH₂)₄₋₆ chain in which one of the CH₂ groups can be replaced by oxygen, sulfur or NR(6), where at least one CH₂ group must stand between the N atom of the NR(4)R(5) and the oxygen, sulfur or NR(6);
or
R(4), R(5) and R(6) independently of one another are hydrogen, (C₁-C₆)-alkyl, (C₃-C₆)-cycloalkyl, or (C₃-C₆)-alkenyl;
E is oxygen or sulfur;
Y is a hydrocarbon chain -[CR(7)₂]ₙ-;
R(7) is H or (C₁-C₂)-alkyl;
n is 1, 2, 3 or 4;
R(3) is a heterocyclic radical of the formula 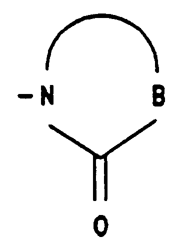
B is (C₃-C₆)-alkenylene, which is unsubstituted or substituted by up to 3 (C₁-C₄)-alkyl groups or by a phenyl radical;
or a physiologically acceptable salt thereof.

2. A compound of the formula I as claimed in claim 1, in which:
R(1) is (C₁-C₄)-alkyl, (C₃-C₅)-cycloalkyl or C_{c}H_{2c}-C_{d}F_{2d+1};
c is 0, 1, 2 or 3;
d is 1, 2, 3, 4, 5 or 6;
R(2) is F, Cl, (C₁-C₆)-alkyl, (C₁-C₆)-alkoxy, (C₁-C₆)-mercaptoalkyl, (C₁-C₆)-fluoroalkoxy, (C₁-C₆)-fluoroalkyl, (C₃-C₆)-cycloalkyl or NR(4)R(5);
R(4) and R(5) together are a (CH₂)₄₋₆ chain, in which one of the CH₂ groups can be replaced by oxygen, sulfur or NR(6), where at least one CH₂ group must stand between the N atom of the NR(4)R(5) and the oxygen, sulfur or NR(6);
or
R(4), R(5) and R(6) independently of one another are hydrogen, (C₁-C₆)-alkyl, (C₃-C₆)-cycloalkyl, or (C₃-C₆)-alkenyl;
or a pyshiologically acceptable salt thereof.

3. A compound of the formula I as claimed in claim 1 and/or claim 2, in which:
R(1) is (C₁-C₄)-alkyl, (C₃-C₄)-cycloalkyl or (C₁-C₄)-fluoroalkyl;
R(2) is F, Cl, (C₁-C₄)-alkyl, (C₁-C₄)-fluoro-alkyl or (C₃-C₆)-cycloalkyl;
Y is a hydrocarbon chain -[CR(7)₂]ₙ-;
R(7) is H or (C₁-C₂)-alkyl;
n is 2 or 3;
or a physiologically acceptable salt thereof.

4. A compound of the formula I as claimed in claim 1 and/or claim 2, in which:
R(1) is (C₁-C₄)-alkyl, (C₃-C₄)-cycloalkyl or (C₁-C₄)-fluoroalkyl;
R(2) is (C₁-C₄)-alkoxy, (C₁-C₄)-mercaptoalkyl or (C₁-C₄)-fluoroalkoxy;
Y is a hydrocarbon chain -[CR(7)₂]ₙ-;
R(7) is H or (C₁-C₂)-alkyl;
n is 2 or 3;
R(3) is a heterocyclic radical of the formula 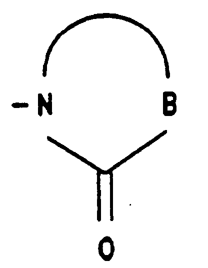
B is (C₃-C₄)-alkenylene, which is unsubstituted or substituted by one to three (C₁-C₄)-alkyl groups or by a phenyl radical;
or a physiologically acceptable salt thereof.

5. A compound of the formula I as claimed in claim 1 and/or claim 2, in which:
R(1) is (C₁-C₄)-alkyl, or (C₁-C₄)-fluoroalkyl;
R(2) is NR(4)R(5);
R(4) and R(5) together are a (CH₂)₄₋₆ chain, in which one of the CH₂ groups can be replaced by oxygen, sulfur or NR(6), where at least one CH₂ group must stand between the N atom of the NR(4)R(5) and the oxygen, sulfur or NR(6);
or
R(4) and R(5) independently of one another are CH₃, C₂H₅, n-C₃H₇, iso-C₃H₇ or cyclo-C₃H₅;
R(6) is H, CH₃ or C₂H₅;
Y is a hydrocarbon chain -[CR(7)₂]ₙ-;
R(7) is H or (C₁-C₂)-alkyl;
n is 2 or 3;
R(3) is a heterocyclic radical of the formula 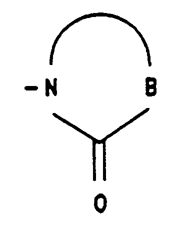
B is (C₃-C₄)-alkenylene, which is unsubstituted or substituted by up to 3 (C₁-C₄)-alkyl groups or by a phenyl radical;
or a physiologically acceptable salt thereof.

6. A process for the preparation of a compound of the formula I as claimed in one or more of the claims 1 to 5, which comprises
(a) reacting a sulfonamide of the formula II or a salt thereof of the formula III 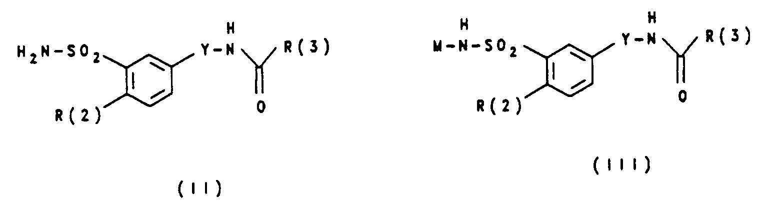 in which M is the cation of an alkali metal or alkaline earth metal, with an R(1)-substituted isocyanate of the formula IV
R(1) - N = C = O (IV)
to give a substituted benzenesulfonylurea of the formula Ia 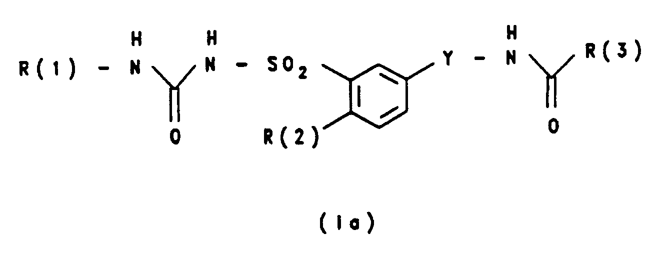 or
(b) preparing a benzenesulfonylurea of the formula Ia from an aromatic benzenesulfonamide of the formula II or a salt thereof of the formula III and an R(1)-substituted trichloroacetamide of the formula V 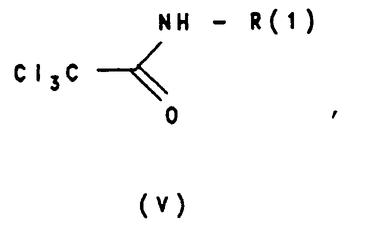 or
(c) preparing a benzenesulfonylthiourea of the formula Ib 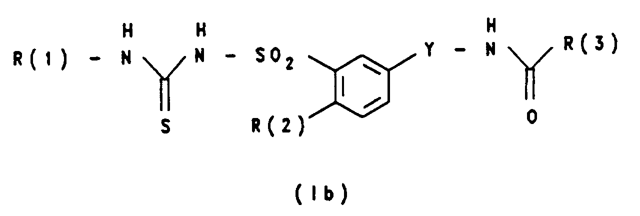 from a benzenesulfonamide of the formula II or a salt thereof of the formula III and an R(1)-substituted thioisocyanate of the formula VI
R(1) - N = C = S (VI)
or
(d) preparing a substituted benzenesulfonylurea of the formula Ia by a conversion reaction from a benzenesulfonylthiourea of the formula Ib, or
(e) preparing a benzenesulfonylurea of the formula Ia by reaction of an amine of the formula R(1)-NH₂ with a benzenesulfonylisocyanate of the formula VII 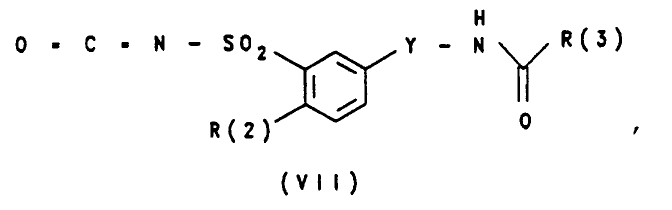 or
(f) preparing a benzenesulfonylthiourea of the formula Ib by reaction of an amine of the formula R(1)-NH₂ with a benzenesulfonylisothiocyanate of the formula VIII

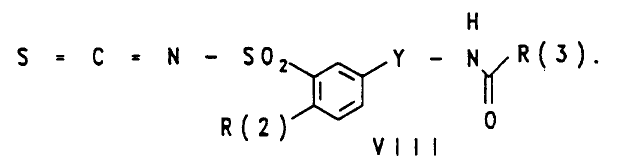

7. The use of a compound of the formula I as claimed in one or more of claims 1 to 5 and/or of a physiologically acceptable salt thereof for the preparation of a medicament for the treatment of disturbances in cardiac rhythm.

8. The use of a compound of the formula I as claimed in one or more of claims 1 to 5 and/or of a physiologically acceptable salt thereof for the preparation of a medicament for prevention of sudden cardiac death.

9. The use of a compound of the formula I as claimed in one or more of claims 1 to 5 and/or of a physiologically acceptable salt thereof for the preparation of a medicament for the treatment of ischemic conditions of the heart.

10. The use of a compound of the formula I as claimed in one or more of claims 1 to 5 and/or of a physiologically acceptable salt thereof for the preparation of a medicament for the treatment of weakened cardiac force.

11. The use of a compound of the formula I as claimed in one or more of claims 1 to 5 and/or of a physiologically acceptable salt thereof for the preparation of a medicament for improving cardiac function following a heart transplant.

12. A medicine comprising an active amount of a compound of the formula I as claimed in one or more of claims 1 to 5 and/or of a physiologically acceptable salt thereof.

## Revendications

1. Benzènesulfonylurées et -thio-urées substituées de formule I 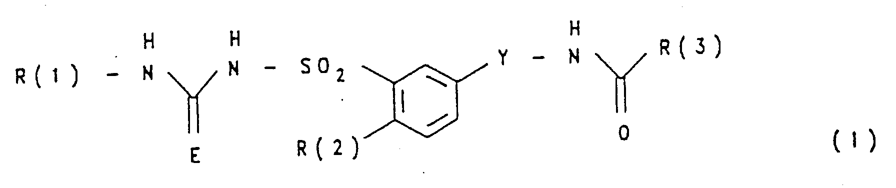 dans laquelle
R(1) représente un groupe alkyle en C₁-C₆, cycloalkyle en C₃-C₆, CₐH₂ₐ-cycloalkyle(C₃-C₅) ou C_{c}H_{2c}-C_{d}F_{2d+1},
a représente 1, 2 ou 3,
c représente 0, 1, 2 ou 3,
d représente 1, 2, 3, 4, 5 ou 6,
R(2) représente F, Cl, Br, I ou un groupe alkyle en C₁-C₆, alcoxy en C₁-C₆, mercaptoalkyle en C₁-C₆, fluoroalcoxy en C₁-C₆, fluoroalkyle en C₁-C₆, cycloalkyle en C₃-C₆ ou NR(4)R(5),
R(4) et R(5) représentant ensemble une chaîne (CH₂)₄₋₆ dans laquelle l'un des groupes CH₂ peut être remplacé par un atome d'oxygène, un atome de soufre ou NR(6), au moins un groupe CH₂ devant être présent entre l'atome d'azote du groupe NR(4)R(5) et l'atome d'oxygène, de soufre ou NR(6),
ou
R(4), R(5), R(6) représentent, indépendamment les uns des autres, un atome d'hydrogène ou un groupe alkyle en C₁-C₆, cycloalkyle en C₃-C₆ ou alcényle en C₃-C₆,
E représente un atome d'oxygène ou de soufre,
Y représente une chaîne hydrocarbonée -[CR(7)₂]ₙ-
R(7) représentant H ou un groupe alkyle en C₁-C₂,
n représentant 1, 2, 3 ou 4,
R(3) représente un hétérocycle de formule 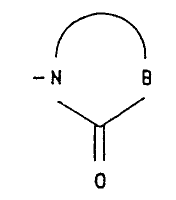
B représentant un groupe alcénylène en C₃-C₆ qui est non substitué ou substitué par jusqu'à 3 groupes alkyle en C₁-C₄ ou par un radical phényle,
et leurs sels physiologiquement acceptables.

2. Composés de formule I selon la revendication 1, dans lesquels
R(1) représente un groupe alkyle en C₁-C₄, cycloalkyle en C₃-C₅ ou C_{c}H_{2c}-C_{d}F_{2d+1},
c représente 0, 1, 2 ou 3,
d représente 1, 2, 3, 4, 5 ou 6,
R(2) représente F, Cl ou un groupe alkyle en C₁-C₆, alcoxy en C₁-C₆, mercaptoalkyle en C₁-C₆, fluoroalcoxy en C₁-C₆, fluoroalkyle en C₁-C₆, cycloalkyle en C₃-C₆ ou NR(4)R(5),
R(4) et R(5) représentant ensemble une chaîne (CH₂)₄₋₆ dans laquelle l'un des groupes CH₂ peut être remplacé par un atone d'oxygène, un atome de soufre ou NR(6), au moins un groupe CH₂ devant être présent entre l'atome d'azote du groupe NR(4)R(5) et l'atome d'oxygène, de soufre ou NR(6),
ou
R(4), R(5), R(6) représentent, indépendamment les uns des autres, un atome d'hydrogène ou un groupe alkyle en C₁-C₆, cycloalkyle en C₃-C₆ ou alcényle en C₃-C₆,
et leurs sels physiologiquement acceptables.

3. Composés de formule I selon la revendication 1 et/ou la revendication 2, dans lesquels
R(1) représente un groupe alkyle en C₁-C₄, cycloalkyle en C₃-C₄ ou fluoroalkyle en C₁-C₄,
R(2) représente F, Cl ou un groupe alkyle en C₁-C₄, fluoroalkyle en C₁-C₄ ou cycloalkyle en C₃-C₆,
Y représente une chaîne hydrocarbonée -[CR(7)₂]ₙ-
R(7) représentant H ou un groupe alkyle en C₁-C₂,
n représentant 2 ou 3,
et leurs sels physiologiquement acceptables.

4. Composés de formule I selon la revendication 1 et/ou la revendication 2, dans lesquels
R(1) représente un groupe alkyle en C₁-C₄, cycloalkyle en C₃-C₄ ou fluoroalkyle en C₁-C₄,
R(2) représente un groupe alcoxy en C₁-C₄, mercaptoalkyle en C₁-C₄ ou fluoroalcoxy en C₁-C₄,
Y représente une chaîne hydrocarbonée -[CR(7)₂]ₙ-
R(7) représentant H ou un groupe alkyle en C₁-C₂,
n représentant 2 ou 3,
R(3) représente un hétérocycle de formule 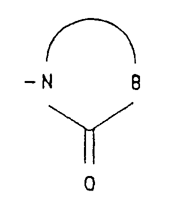
B représentant un groupe alcénylène en C₃-C₆ qui est non substitué ou substitué par 1 à 3 groupes alkyle en C₁-C₄ ou par un radical phényle,
et leurs sels physiologiquement acceptables.

5. Composés de formule I selon la revendication 1 et/ou la revendication 2, dans lesquels
R(1) représente un groupe alkyle en C₁-C₄ ou fluoroalkyle en C₁-C₄,
R(2) représente NR(4)R(5),
R(4) et R(5) représentant ensemble une chaîne (CH₂)₄₋₆ dans laquelle l'un des groupes CH₂ peut être remplacé par un atome d'oxygène, un atome de soufre ou NR(6), au moins un groupe CH₂ devant être présent entre l'atome d'azote du groupe NR(4)R(5) et l'atome d'oxygène, de soufre ou NR(6),
ou
R(4), R(5) représentent, indépendamment l'un de l'autre, le groupe CH₃, C₂H₅, n-C₃H₇, iso-C₃H₇ ou cyclo-C₃H₅,
R(6) représente H, CH₃ ou C₂H₅,
Y représente une chaîne hydrocarbonée -[CR(7)₂]ₙ-
R(7) représentant H ou un groupe alkyle en C₁-C₂,
n représentant 2 ou 3,
R(3) représente un hétérocycle de formule 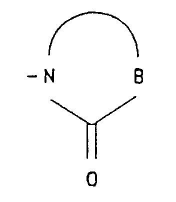
B représentant un groupe alcénylène en C₃-C₄ qui est non substitué ou substitué par jusqu'à 3 groupes alkyle en C₁-C₄ ou par un radical phényle,
et leurs sels physiologiquement acceptables.

6. Procédé pour la préparation d'un composé de formule I selon une ou plusieurs des revendications 1 à 5, caractérisé en ce que
(a) on fait réagir un sulfonamide de formule II ou un de ses sels de formule III 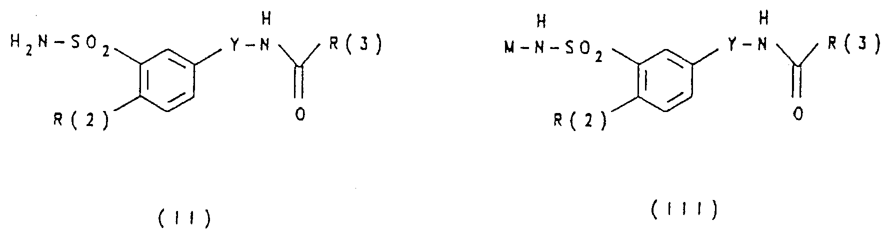 dans laquelle M est le cation d'un métal alcalin ou alcalino-terreux,
avec un isocyanate substitué par R(1), de formule IV
R(1)-N=C=O IV
pour aboutir à une benzènesulfonylurée substituée de formule Ia, 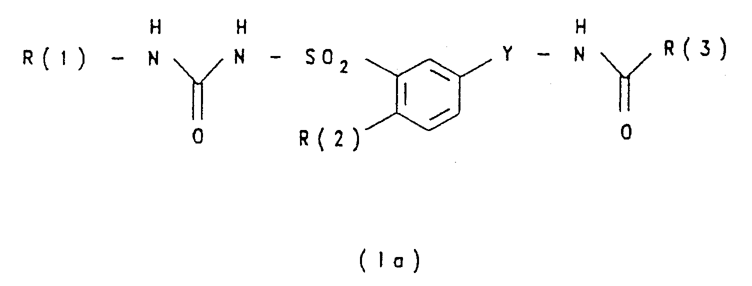 ou en ce que
(b) on prépare une benzènesulfonylurée de formule Ia à partir d'un benzènesulfonamide aromatique de formule II ou d'un de ses sels de formule III et d'un trichloracétamide substitué par R(1), de formule V 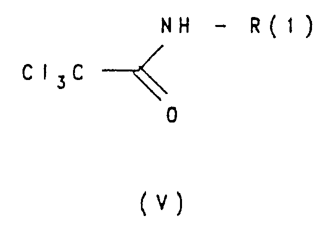 ou en ce que
(c) on prépare une benzènesulfonylthio-urée de formule Ib 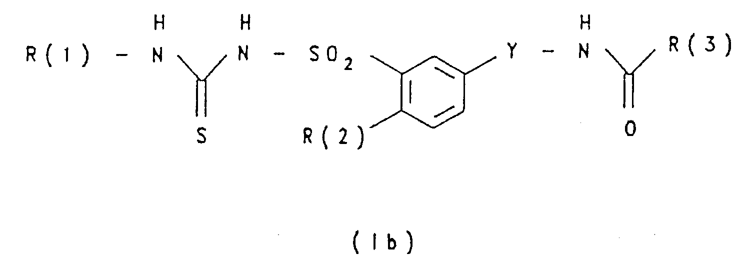 à partir d'un benzènesulfonamide de formule II ou d'un de ses sels de formule III et d'un thio-isocyanate substitué par R(1) de formule VI
R(1)-N=C=S VI
ou en ce que
(d) on prépare une benzènesulfonylurée substituée de formule Ia par une réaction de conversion à partir d'une benzènesulfonylthio-urées de formule Ib,
ou en ce que
(e) on prépare une benzènesulfonylurée de formule Ia en faisant réagir une amine de formule R(1)-NH₂ avec un isocyanate de benzènesulfonyle de formule VII 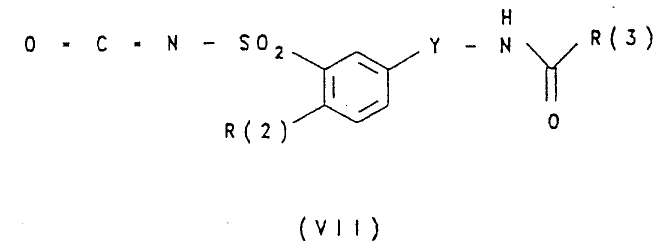 ou en ce que
(f) on prépare une benzènesulfonylthio-urées de formule Ib en faisant réagir une amine de formule R(1)-NH₂ avec un isothiocyanate de benzènesulfonyle de formule VIII

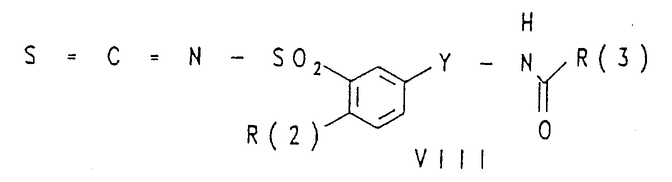

7. Utilisation d'un composé de formule I selon une ou plusieurs des revendications 1 à 5 et/ou d'un sel physiologiquement acceptable de celui-ci, pour la fabrication d'un médicament destiné au traitement de troubles du rythme cardiaque.

8. Utilisation d'un composé de formule I selon une ou plusieurs des revendications 1 à 5 et/ou d'un sel physiologiquement acceptable de celui-ci, pour la fabrication d'un médicament destiné à empêcher la mort subite par arrêt cardiaque.

9. Utilisation d'un composé de formule I selon une ou plusieurs des revendications 1 à 5 et/ou d'un sel physiologiquement acceptable de celui-ci, pour la fabrication d'un médicament destiné au traitement d'états ischémiques du coeur.

10. Utilisation d'un composé de formule I selon une ou plusieurs des revendications 1 à 5 et/ou d'un sel physiologiquement acceptable de celui-ci, pour la fabrication d'un médicament destiné au traitement de la force cardiaque affaiblie.

11. Utilisation d'un composé de formule I selon une ou plusieurs des revendications 1 à 5 et/ou d'un sel physiologiquement acceptable de celui-ci, pour la fabrication d'un médicament destiné à l'amélioration de la fonction cardiaque après une greffe du coeur.

12. Médicament, caractérisé par une quantité efficace d'un composé de formule I selon une ou plusieurs des revendications 1 à 5 et/ou d'un sel physiologiquement acceptable de celui-ci.
